# EUROPEAN PATENT SPECIFICATION

(11) **EP 1 712 247 B1**
(45) Date of publication and mention of the grant of the patent: **04.03.2020**
(21) Application number: 06007502.5
(22) Date of filing: 10.04.2006
(51) Int. Cl.: A61M 25/00

(54) **Catheter**
Katheter
Cathéter

(30) Priority: 11.04.2005 JP 2005112927
(43) Date of publication of application: 18.10.2006
(60) Divisional application: 20151323.1
(73) Proprietor: Terumo Kabushiki Kaisha, Tokyo (JP)
(72) Inventor: Itou, Takenari, Fujinomiya-city Shizuoka (JP); Fukuoka, Tetsuya, Fujinomiya-city Shizuoka (JP)
(74) Representative: Casalonga, Axel

(56) References cited:
- EP-A- 0 807 444
- EP-A- 1 344 549
- US-A- 5 700 253
- US-A- 5 891 114
- US-A1- 2004 176 740

## Description

The present invention generally relates to a catheter; insertible into a blood vessel. More particularly, the invention pertains to a guiding catheter for guiding a PTCA catheter or the like to a target location.

A guiding catheter is a catheter that is used to guide, for example, a PTCA catheter to a target location to effect therapy of a coronary artery of a heart.

One requirement associated with guiding catheters is that they possess a relatively smaller outside diameter to reduce the incision at the place of insertion info a blood vessel and to reduce the friction between the catheter and the blood vessel, to thereby alleviate the burden on the patient. On the other hand, the PTCA catheter (a dilation catheter, a stent conveying catheter or the like) used for therapy is required to be relatively larger in size in order to display a sufficient effect at the portion to be treated. This requirement demands that the guiding catheter possess a relatively large inside diameter.

Generally speaking, in recent years the outside diameter of guiding catheters have typically been 6 Fr (2.06 mm) and 7 Fr (2.36 mm), for example. When the outside diameter is smaller, the invasiveness to the patient is reduced, but the surgical procedure becomes more difficult. Assuming a fixed outside diameter of the catheter, the catheter tube wall tends to be weakened and the probability of collapse or kinking enhanced when the thickness of the catheter tube wall is reduced. To address this problem, it may be contemplated to make the catheter shaft portion flexible so as to enhance the kink resistance, or to reduce the inside diameter so as to enlarge the material thickness of the tube wall and enhance the kink resistance.

Generally speaking, the kink resistance increases as the catheter shaft portion is made more flexible. However, a highly flexible catheter shaft portion has the problem that, since it is quite difficult or perhaps impossible to obtain a high pushability (i.e., a capability to transmit a pushing force) at the time of insertion into a blood vessel, it is difficult to pass the catheter in a meandering blood vessel. In addition, where a distal end portion provided with a curved shape is too soft, there is the problem that the catheter distal end would be easily disengaged from a coronary ostium by a device operation such as insertion of a PTCA catheter after the catheter distal end is engaged with the coronary ostium from the aorta.

On the other hand, if the catheter is simply made stiff, the catheter van easily break and the kink resistance thereof is not enhanced. In addition, the curved shape portion is passed through a guiding sheath in use, or is inserted into a blood vessel via a guiding sheath after the curved shape portion is put into a straight form by passing a guide wire in the catheter lumen. In this case, if the straightened shape does not quickly return to the original shape before the coronary artery upon the evulsion of the guide wire, the curved shape portion cannot be engaged with the coronary ostium, and too large a backup force also leads to an inconvenience.

United States Patent Nos. 6,042,578 and 5,755,704 set forth proposals intended to enhance the performances of guiding catheters. At present, however, there has not been proposed a guiding catheter possessing sufficiently desirable physical properties such as rigidity and kink resistance.

United States Patent No. 6,042,578 discloses a catheter in which the sizes of a reinforcing braid of a catheter are so set that a radiopacity performance can be obtained. However, the catheter described in this patent cannot satisfactorily fulfill the above-mentioned physical performances.

United States Patent No. 5,755,704 discloses a catheter in which an inner layer is absent and reinforcing wires are exposed on the inside surface. However, this catheter also does not provide sufficient performance characteristics such as those mentioned above.

Through intensive and extensive studies, a catheter (guiding catheter) has been developed which possesses highly desirable characteristics and performance capabilities such as those discussed above. The catheter possesses a shaft portion which is less liable to kink when curved according to the shape of a blood vessel and is sufficiently rigid as to relatively easily pass through a sharply bent blood vessel, yet also possesses an inside diameter that is relatively large in comparison to the outside diameter.

The catheter comprises an elongated tubular body possessing an outside diameter of from 1.35 to 3 mm, with the tubular body comprising a distal end portion and a proximal end portion, and being comprised of an inner layer forming an inside surface of the tubular body, an outer layer forming an outside surface of the tubular body, and a plurality of reinforcing wires between the inside surface and the outside surface. The reinforcing wires each possess a length, a thickness substantially parallel to a radial direction of the tubular body and a width substantially perpendicular to the thickness. The ratio of the wall thickness of the tubular body to the thickness of the reinforcing wires is from 3.5 to 3.8, and the proportion of the total cross-sectional area of the plurality of reinforcing wires to the cross-sectional area of the tubular body is less than 25%, but not less than 17%.

According to a preferred embodiment, the ratio of the width to the thickness of the reinforcing wires is more than 2.5 and less than 3.6, and the ratio of the outer circumference of the tubular body to the width of the reinforcing wires is from 54 to 61.6.

According to other preferred aspects, the ratio of the outside diameter of the tubular body to the thickness of the reinforcing wires is from 55 to 65 and the ratio of the inside diameter to the outside diameter of the tubular body is from 0.85 to 0.91.

In addition, the catheter preferably possesses a kink resistance (length) of not more than 20 mm as measured by the loop method. The kink resistance measured by the loop method is preferably not more than 15 mm, and more preferably not more than 10 mm. Here, the kink resistance measured by the loop method is the length measured as follows. A 10 mm thick plate is provided with two through-holes having a diameter of 2.8 mm, with a center-to-center distance being 10 mm. With the plate immersed in water at 37°C, the catheter is passed through the through-holes, and is curved to form a loop. One end of the catheter is pulled, and at the time when kinking occurs, the length from the plate to the loop is measured as an indication of kink resistance. Namely, kink resistance is better as the length is smaller.

According to another aspect, a catheter comprises an elongated tubular body possessing an inside diameter of from 1.2 to 2.85 mm, with the tubular body comprising a distal end portion and a proximal end portion, and being comprised of an inner layer forming an inside surface of the tubular body, an outer layer forming an outside surface of the tubular body, and a plurality of reinforcing wires between the inside surface and the outside surface. The reinforcing wires each possess a length, a thickness substantially parallel to a radial direction of the tubular body and a width substantially perpendicular to the thickness. The ratio of the wall thickness of the tubular body to the thickness of the reinforcing wires is from 3.5 to 3.8, and the proportion of the total cross-sectional area of the plurality of reinforcing wires to the cross-sectional area of the tubular body is less than 25%, but not less than 17%.

In accordance with another aspect, a catheter comprises an elongated tubular body possessing an inside diameter and an outside diameter, with the tubular body possessing at least one of an outside diameter of from 1.35 to 3 mm and an inside diameter of from 1.2 to 2.85 mm, and with the ratio of the inside diameter of the tubular body to the outside diameter of the tubular body being from 0.85 to 0.91. The tubular body comprises a distal end portion and a proximal end portion, and is comprised of an inner layer forming an inside surface of the tubular body, an outer layer forming an outside surface of the tubular body, and a plurality of reinforcing wires between the inside surface and the outside surface. The reinforcing wires each possess a length, a thickness substantially parallel to a radial direction of the tubular body and a width substantially perpendicular to the thickness. The ratio of the wall thickness of the tubular body to the thickness of the reinforcing wires is from 3.5 to 3.8, and the proportion of the total cross-sectional area of the plurality of reinforcing wires to the cross-sectional area of the tubular body is less than 25%, but not less than 17%.

The above and additional features and characteristics of the present invention will become apparent from the following description, considered together with the accompanying drawing figures in which like elements are designated by like reference characters.
Fig. 1 is a side view of an embodiment of a catheter tube as disclosed herein.
Fig. 2 is a longitudinal cross-sectional view of the catheter tube shown in Fig. 1.
Fig. 3 is a transverse cross-sectional view of the catheter tube shown in Fig. 1.
Fig. 4 shows the reinforcing wires used in the catheter tube of Fig. 1.
Fig. 5 is a schematic illustration of a kink resistance evaluation test.
Fig. 6 is a schematic illustration of a flexural rigidity evaluation test.
Fig. 7 is a schematic illustration of a collapse strength evaluation test.
Fig. 8 is a schematic illustration of a distal end shape restoring performance evaluation test.
Fig. 9 is a schematic illustration of a backup force evaluation test.

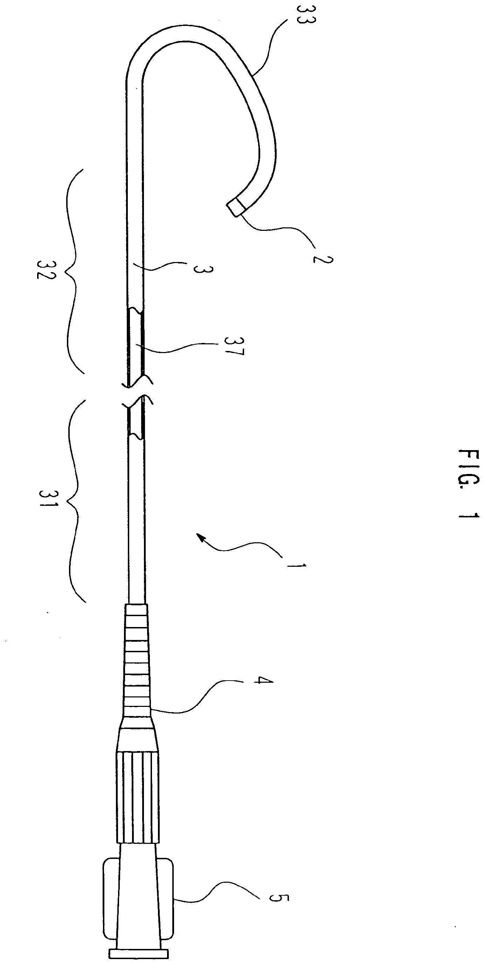

The catheter 1 shown in Fig. 1 has useful application as a guiding catheter for guiding a treatment catheter (device), for example, a dilation catheter for PTCA (balloon catheter), a catheter for conveying a stent in a radially contracted state to a stenosis portion, radially expanding the stent to indwell at the stenosis portion so as to dilate the stenosis portion and maintain the stenosis portion in the dilated state (stent conveying catheter), or the like to a target location such as a stenosis portion of a coronary artery.

The catheter 1 is comprised of a catheter main body 3, a soft tip 2 that exhibits relatively high flexibility and is mounted on the distal side or distal end of the catheter main body 3, and a hub 5 (catheter hub) provided on the proximal side or proximal end of the catheter main body 3. In addition, a cover member 4 serving as an anti-kinking protector 4 and formed of an elastic material is provided at a position for connection between the catheter main body 3 and the hub 5. This cover member 4 prevents the catheter 1 from sharply bending (kinking) in the vicinity of the connection portion.

The catheter main body 3 is comprised of a flexible tubular body, and is provided in its generally central portion with a lumen 37 extending over the entire length of the catheter main body 3. The lumen 37 opens to the distal end of the soft tip 4.

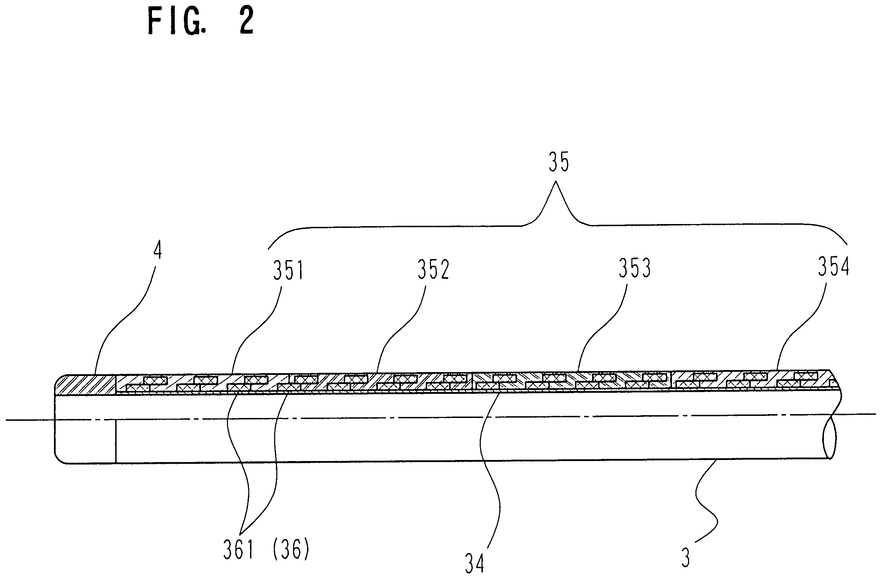

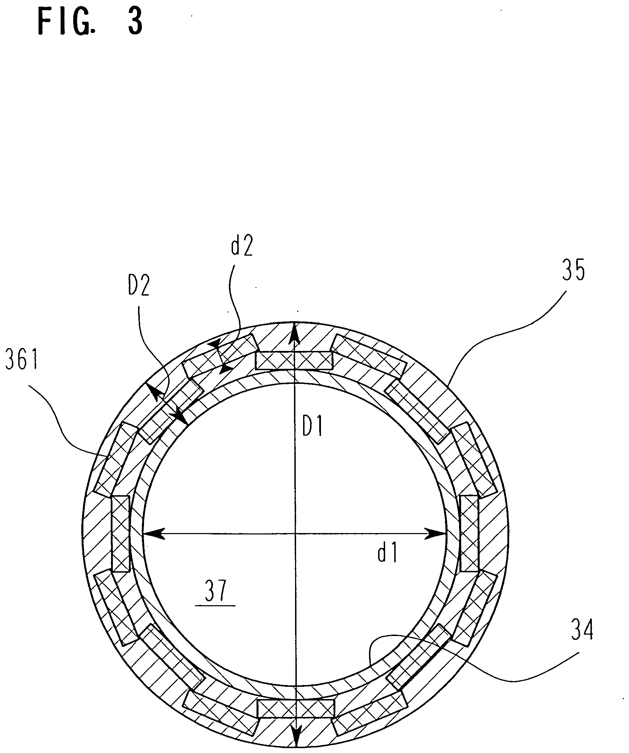

As shown in Fig. 2, the tubular body constituting the catheter main body 3 is comprised of a laminate of three layers. The three layers include an inner layer 34 defining the inside surface of the tubular body, an outer layer 35 defining the outside surface of the tubular body, and a reinforcement layer 36 located between the inner and outer surfaces defines by the inner and outer layers 34, 35 as shown in Fig. 3. The reinforcement layer 36 is composed of a plurality of reinforcing wires. The gaps between the plurality of reinforcing wires in the reinforcement layer 36 are filled with the resin of the outer layer 35 and/or the inner layer 34, and the reinforcing wires are embedded in one or both of the resin layers.

The outer layer 35 has a first region 351, a second region 352 located on the proximal side of the first region 351, a third region 353 located on the proximal side of the second region 352, and a fourth region 354 located on the proximal side of the third region 353. The third region 353 is more flexible than the fourth region 354, the second region 352 is more flexible than the third region 353, and the first region 351 is more flexible than the second region 352. This imparts characteristics to the catheter main body so that the catheter main body 3 gradually increases in flexibility along the distal direction. At the time of inserting the catheter 1 into a blood vessel, it is thus possible to insert the catheter 1 to the blood vessel with a higher degree of safety and less risk of damage to the patient, while also imparting characteristics to the catheter providing sufficient pushability and distal torque transmission performance.

Examples of the material used in the fabrication of each of the first region 351, the second region 352, the third region 353 and the fourth region 354 include various thermoplastic elastomers based on styrene, polyolefin, polyurethane, polyester, polyamide, polybutadiene, transpolyisoprene, fluoro-rubber, chlorinated polyethylene or the like, which may be used either singly or in combination of two or more thereof (polymer alloys, polymer blends, laminates, etc.).

The material constituting the inner layer 34 is preferably a material selected so that, at the time of inserting a device such as a treatment catheter and a guide wire in the lumen 37 of the catheter main body 3, at least the portion coming into contact with the device exhibits relatively low friction. This helps ensure that the device inserted in the catheter main body 3 can be moved or inserted in the longitudinal direction under a relatively lower sliding resistance, contributing to enhancement of the operational characteristics. Naturally, the inner layer 34 may be entirely composed of a relatively low-friction material.

Examples of low-friction material in this case include fluoro-resin materials such as polytetrafluoroethylene (PTFE).

The reinforcement layer 36 includes reinforcement comprised of a plurality of reinforcing wires or filamentous members 361 for reinforcing the catheter main body 3. Examples of the reinforcement include filamentous members 361 set into a spiral form or a net-like form. The filamentous members 361 are preferably made of a metal such as stainless steel. More specific examples include those formed by a method in which stainless filaments are pressed down into a flat plate-like shape, and a plurality (about 8 to 32) of such flat plate-like filaments are put into a spiral form or braided (braid) so that the wall thickness of the catheter main body 3 can be relatively thin in the radial direction. The number of filamentous members 361 used here is preferably a multiple of eight, for achieving a balanced reinforcement of the catheter main body 3 in the tubular shape.

The provision of the reinforcement layer 36 as described above makes it possible to achieve a desirable degree of rigidity and strength, without increasing the wall thickness of the catheter main body 3 (i.e., while maintaining the inside diameter (the diameter of the lumen 37) comparatively large). As a result, the catheter 1 permits the passage therethrough of the PTCA catheter or the like having a comparatively large outside diameter. At the same time, the catheter 1 possesses excellent pushability characteristics and torque transmission performance, while also being less susceptible to kinking or collapse.

It is to be understood that the number of layers constituting the catheter main body 3, the constituent materials of the layers, the presence or absence of the reinforcement, and other factors, may vary along the longitudinal extent of the catheter main body 3. For example, to further enhance the flexibility of the distal side portion (e.g., the distal end portion 33) of the catheter main body 3, the number of layers in such portion may be reduced, a more flexible material may be used to constitute such portion, or the reinforcement may be absent only at such portion.

Since the insertion of the catheter 1 into a living body may be carried out while confirming the position of the catheter under radioscopic observation, a radiopaque material (radioscopic contrast agent) is preferably blended in the material forming the outer layer 35. Examples of the radiopaque material which can be used here include barium sulfate, bismuth oxide, and tungsten. Further, the radiopaque material is preferably blended in the material forming the outer layer 35 in a proportion of from 30 to 80 wt%.

In addition, the radiopaque material may not necessarily be present over the whole length of the catheter main body 3. That is, the radiopaque material may be present in only a part of the catheter main body 3, for example, only in the distal end portion 33, or only in the soft tip 2.

The catheter main body 3 possesses, in order from the proximal end side along the longitudinal extent of the main body, a proximal portion 31 and an intermediate portion 32 which extend substantially rectilinearly, and a distal end portion or curved portion 33 extending further in the distal direction from the intermediate portion 32. The distal end portion possesses a desired curved shape. The distal end portion 33 is curved in a desired shape suited to the portion into which the distal end portion 33 of the catheter main body 3 is to be inserted, such as the left coronary artery and the right coronary artery. Particularly, the distal end portion 33 has such a shape as to facilitate the operation of engaging the distal end portion 33 with the coronary ostium (engaging operation) or such a shape as to make it possible to maintain the distal end portion 33 in engagement with the coronary ostium more securely.

With respect to the first region 351, the second region 352, the third region 353 and the fourth region 354 described above, at least the first region 351 is preferably formed or provided at the distal end portion 33. In the preferred embodiment, the distal end portion 33 further includes the second region 352, third region 353 and fourth region 354.

In addition, the soft tip 2 is attached to the distal end of the distal end portion (curved portion) 33. The soft tip 2 is composed of a material rich in flexibility, with the distal end thereof preferably possessing a rounded shape. The soft tip 2 is thus constructed to facilitate smooth and safe movement even in a blood vessel which is curved, sharply bent, or branched. Examples of the material constituting the soft tip 2 include various rubber materials such as natural rubber, isoprene rubber, butadiene rubber, chloroprene rubber, silicone rubbers, fluoro-rubbers, styrene-butadiene rubber, etc., and various thermoplastic elastomers based on styrene, polyolefin, polyurethane, polyester, polyamide, polybutadiene, transpolyisoprene, fluoro-rubber, chlorinated polyethylene, or the like.

The above-mentioned radiopaque material (radioscopic contrast agent) may be blended in the constituent material of the soft tip 2.

The length of the soft tip 2 is not particularly limited. However, in general, the length of the soft tip 2 is preferably about 0.5 to 3 mm, more preferably about 1 to 2 mm.

The hub 5 is attached (fixed) to the proximal end of the catheter main body 3. The hub 5 is provided with an inner cavity communicated with the lumen 37. The inner cavity has an inside diameter approximately equal to the inside diameter of the lumen 37 so that the inner cavity is continuous with the inside surface of the proximal end portion of the lumen 37 without any step or the like therebetween.

Long bodies (filamentous bodies) such as a guide wire, catheters (e.g., a PTCA balloon catheter or stent conveying catheter), an endoscope, an ultrasonic probe, a temperature sensor, etc. are adapted to be inserted or evulsed through the hub 5. Various liquids such as contrast agent (radioscopic contrast agent), liquid chemicals, physiological saline, etc. can also be fed in. In addition, the hub 5 may be connected to other implements, such as a Y-type branch connector.

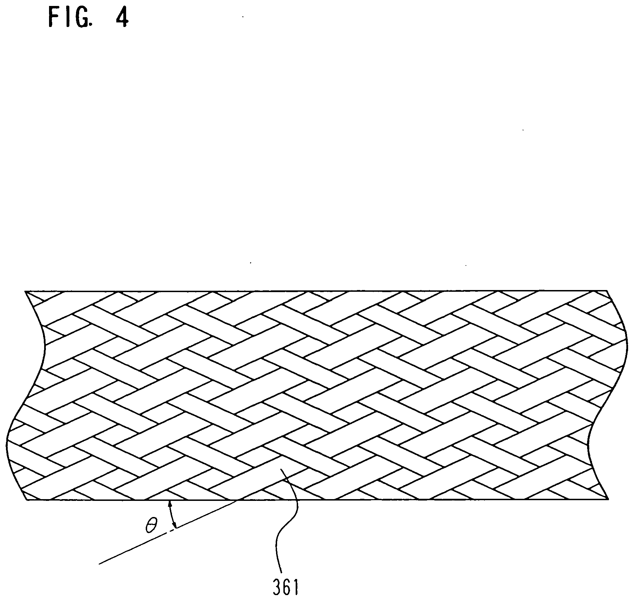

Preferable sizes of component parts of the catheter main body 3 in this embodiment will now be described below, referring to Figs. 3 and 4.

The outside diameter D1 of the catheter main body 3 is preferably from 1.35 to 3 mm. If the outside diameter D1 is too large, the operational characteristics and ability will be lowered and the burden on the patient will be increased such as when inserting and moving the catheter main body 3 in an artery.

In addition, the inside diameter d1 of the catheter main body 3 is preferably from 1.2 to 2.85 mm. If the inside diameter d1 is too small, the outside diameter of a treatment catheter or the like that can be inserted in the catheter main body 3 is reduced accordingly, and the choice of devices with which the main body can be used is undesirably limited.

With the outside diameter and the inside diameter of the catheter main body 3 represented as D1 mm and d1 mm respectively, the ratio d1/D1 of the inside diameter to the outside diameter is 0.85 - 0.91, preferably from 0.87 to 0.91. If the ratio d1/D1 is too small, the wall thickness of the catheter main body 3 is enlarged accordingly, and the inside diameter is reduced accordingly, so that the devices which can be guided into the catheter main body 3 are limited. On the other hand, if the ratio d1/D1 is too large, a sufficient wall thickness of the catheter main body 3 may not be obtained, the backup force is weakened, and kink resistance in use is lowered. The term "backup" means that a guiding catheter engaged with the coronary ostium supports a PTCA catheter or the like in a lumen of the guiding catheter here. The phrase "backup force" means the force to keep in the engaging (fixing) state and not to disengage from the coronary ostium when the PTCA catheter or the like advances to the depth of the coronary here.

The thickness of the inner layer 34 is preferably from 8 to 20 *µ*m. The thickness is desirably selected to ensure even covering of the inside surface of the catheter main body 3 and is desirably as small as possible.

The filamentous members (reinforcing wires) 361 constituting the reinforcement layer 36 are so formed that a plurality of them are wound around the surface of the inner layer 34. The sizes of the filamentous members 361 vary depending on the outside diameter of the catheter main body 3 and the number of filamentous members used, and are so designed that the ratio D2/d2 of the wall thickness D2 of the catheter main body 3 and the size (thickness) d2 of the filamentous members 361 in the radial direction of the catheter main body 3 is from 3.5 to 3.8. In addition, the proportion of the total sectional area of the plurality of the filamentous members 361 relative to the cross-sectional area of the catheter main body 3 (the cross-sectional area in the direction perpendicular to the longitudinal direction of the catheter main body 3) is not less than 17%, but is less than 25%. If the ratio D2/d2 is in excess of 3.8, the flexibility of the catheter main body 3 is undesirably impacted or spoiled. On the other hand, if the ratio D2/d2 is below 3.5, the kink resistance will be unsatisfactory. Further, if the proportion of the total sectional area of the plurality of the filamentous members 361 relative to the cross-sectional area of the catheter main body 3 is not less than 25%, the flexibility of the catheter main body 3 is negatively affected or spoiled; while if the proportion is less than 17%, the kink resistance of the catheter main body 3 is undesirably impacted or spoiled.

As an embodiment typically fulfilling the above-mentioned conditions, a preferable configuration can be employed in which the filamentous members 361 are rectangular in section, with the side (width) along the surface of the inner layer 34 being the longer side of the rectangle and the side (thickness) in the radial direction of the catheter main body 3 being the shorter side of the rectangle. While a preferred cross-sectional shape of the filamentous members 361 is roughly rectangular, it suffices that the sides of the cross-sectional shape in the width direction are roughly parallel and rectilinear; and the left and right sides of the sectional shape in the thickness direction may be slightly bulged. Flat plate-like reinforcing wires receive forces more evenly in the presence of an external stress and will therefore show more constant physical properties, as compared with reinforcing wires which are elliptic in section.

The number of the filamentous members 361 is preferably 16. Where the number of filamentous members 361 is 16, the ratio (width/thickness) of the width to the thickness of the filamentous members 361 is preferably more than 2.5 and less than 3.6, more desirably from 3.1 to 3.4. If the width of the filamentous members 361 is too small, the number of sharp bending points per unit length is increased, and only plastic portions are bent, so that the stress to bending will be small, resulting in a flexible state. On the other hand, if the width is too large, there is not the same sharp bending points, and the reinforcing wires are bent for bending the catheter main body 3. Thus, the catheter main body 3 will be rigid but susceptible to kinking. It has been found that a catheter with the width-to-thickness ratio of the reinforcing wires in the above-mentioned range possesses relatively high flexural rigidity, is relatively hard, and possesses excellent kink resistance capability.

The width of the filamentous members 361 is preferably from 110 to 126 *µ*m, and the thickness of the filamentous members 361 is preferably from 35 to 40 *µ*m, which is greater than the thickness of the inner layer 34. It should be noted here that, at the time of calculating the proportion of the total sectional area of the plurality of the filamentous members 361 based on or relative to the cross-sectional area of the catheter main body 3, it is necessary to take into account the fact that the filamentous members 361 are wound in a skewed manner against the axial direction of the catheter main body 3, as shown in Fig. 4, and so the apparent width and cross-sectional area of the filamentous members 361 are increased accordingly.

The angle *θ* of the filamentous members 361 relative to the longitudinal direction of the catheter main body 3 is preferably from 65 to 75 degrees, more preferably from 69 to 72 degrees. In this case, it is preferable that all of the filamentous members 361 are wound at the same angle.

The cross-sectional area of the catheter main body 3 is from 0.3 to 1.96 mm², and the total cross-sectional area of the plurality of the filamentous members 361 is from 0.051 to 0.49 mm². The cross-sectional area of each filamentous members is larger than an area of width × thickness of the each filamentous members. Because the filamentous members are aslant arranged to the axial direction of the catheter main body 3 and its width in the cross-section becomes larger than the actual width of the each filamentous members.

In addition, the ratio (outer circumference/width) of the outer circumference of the catheter main body 3 to the width of the filamentous members 361 is preferably from 54 to 61.6. These values all satisfy the conditions that the catheter main body 3 is not too hard and has a satisfactory kink resistance and that an outside diameter as small as possible can be obtained while achieving an inside diameter sufficient for the manual operations (procedure).

In addition, the ratio (outside diameter/thickness) of the outside diameter of the catheter main body 3 to the thickness (the thickness in the radial direction of the catheter main body 3) of the filamentous members 361 is preferably from 55 to 65. Particularly, if the filamentous members 361 are too thick (i.e., the outside diameter/thickness is less than 55), cracks may be generated in the outside surface of the catheter main body 3, and the distal end shape restoring performance may be lowered. On the other hand, if the filamentous members 361 are too thin (i.e., the outside diameter/thickness is in excess of 65), the kink resistance is undesirably lowered.

### Examples

Now, Examples of the present invention and Comparative Examples will be described below.

### Example 1

On a wire member obtained by coating a copper wire having a diameter of 1.80 mm with a 10 *µ*m-thick PTFE layer, stainless steel flat plate-like reinforcing wires (16 wires in a set) of 110 *µ*m width and 35 *µ*m thickness are wound in a braided form at an interval of 0.2 mm.

Both ends of the reinforcing wires are cut, and four short tubes having the same length of 5 mm, but formed of polyester elastomers increased stepwise in hardness in the proximal direction are fitted over the wire member, with the distal-most tube being a 5 mm-long short tube of a polyester elastomer resin having a Shore hardness of 30 D and containing 68 weight % of tungsten as a radioscopic contrast agent and 4 weight % of a pigment. Finally, a polyester elastomer tube having a Shore hardness of 78 D is fitted over the remaining about 950 mm portion on the proximal side of the wire member. The end portions of the tubes are abutted on each other, and the whole body is covered with a heat-shrinkable tube, followed by heating to achieve thermal fusing. Thereafter, the heat-shrinkable tube was peeled off, and the copper wire was drawn out to obtain a tube having an outside diameter of 2.06 mm, an inside diameter of 1.80 mm, an inside diameter/outside diameter ratio of 0.87, and a length of 1000 mm, with a lumen penetrating therethrough. The outside surface of the portion ranging from 30 mm to 950 mm distance from the distal end of the tube was subjected to surface roughening (creping) on a heated plate having a rugged surface.

A distal end soft tip was connected to the tube obtained as above, and was rounded by heating in a metal die, to obtain a catheter main body.

A core metal having a curved shape was placed in the catheter main body completed as above, and the assembly was heated in an oven to deform the catheter main body, whereby the distal end of the catheter main body was formed into the shape of Judkins left 4.0 (the numeral indicates the inner diameter of the aorta to use the catheter in centimeter (cm)). Finally, a hub and an anti-kinking protector were attached to the proximal end side of the catheter main body to obtain a guiding catheter of 6 Fr size.

Of the catheter thus obtained, the width-to-thickness ratio of the reinforcing wires was 3.15, the ratio of the outside diameter of the catheter to the width of the reinforcing wires was 58.9, and the sectional area occupying ratio of the reinforcing wires was 19.0%.

### Example 2

A catheter was produced by the same method as in Example 1, except that the interval between the reinforcing wires was set to 0.15 mm. In the catheter thus obtained, the width-to-thickness ratio of the reinforcing wires was 3.15, the ratio of the outside diameter of the catheter to the width of the reinforcing wires was 58.9, and the cross-sectional area occupying ratio of the reinforcing wires was 24.8%.

### Example 3

A catheter was produced by the same method as in Example 1, except that the interval between the reinforcing wires was set to 0.15 mm, and the tension in winding the reinforcing wires was increased to 2.5 times that in Example 1. In the catheter thus obtained, the width-to-thickness ratio of the braid was 3.15, the ratio of the catheter outside diameter to the width of the reinforcing wires was 58.9, and the sectional area occupying ratio of the reinforcing wires was 24.8%.

### Example 4

On a wire member obtained by coating a copper wire having a diameter of 2.06 mm with a 10 *µ*m-thick PTFE layer, stainless steel flat plate-like reinforcing wires (16 wires in a set) of 126 *µ*m width and 40 *µ*m thickness are wound in a braided form at an interval of 0.2 mm while setting the winding force exerted on the reinforcing wires to 250 gf.

Both ends of the reinforcing wires are cut, and four short tubes having the same length but formed of polyester elastomers increased stepwise in hardness in the proximal direction are fitted over the wire member, with the distal-most tube being a 5 mm-long short tube of a polyester elastomer resin having a Shore hardness of 30 D and containing 68 weight % of tungsten as a radioscopic contrast agent and 4 weight % of a pigment. Finally, a polyester elastomer tube having a Shore hardness of 68 D is fitted over the remaining about 950 mm portion on the proximal side of the wire member. The end portions of the tubes are abutted on each other, and the whole body is covered with a heat-shrinkable tube, followed by heating to achieve thermal fusing. Thereafter, the heat-shrinkable tube was peeled off, and the copper wire was drawn out, to obtain a tube having an outside diameter of 2.36 mm, an inside diameter of 2.06 mm, an inside diameter/outside diameter ratio of 0.87, and a length of 1000 mm, with a lumen penetrating therethrough. The outside surface of the portion ranging from 30 mm to 950 mm distance from the distal end of the tube was subjected to surface roughening (creping) on a heated plate having a rugged surface.

A distal end soft tip was connected to the tube obtained as above, and was rounded by heating in a metal die to obtain a catheter main body.

A core metal having a curved shape was placed in the catheter main body completed as above, and the assembly was heated in an oven to deform the catheter main body, whereby the distal end of the catheter main body was formed into the shape of Judkins left 4.0. Finally, a hub and an anti-kinking protector were attached to the proximal end side of the catheter main body, to obtain a guiding catheter of 7 Fr in size.

In the catheter thus obtained, the width-to-thickness ratio of the reinforcing wires was 3.15, the ratio of the catheter outside diameter to the width of the reinforcing wires was 58.9, and the cross-sectional area occupying ratio of the reinforcing wires was 23.6%.

### Comparative Example 1

A 6 Fr guiding catheter was produced by the same method as in Example 1, except that the reinforcing wires had a thickness of 35 *µ*m and a width of 80 *µ*m. In the catheter thus obtained, the width-to-thickness ratio of the braid was 2.29, the ratio of the catheter outside diameter to the width of the reinforcing wires was 58.9, and the cross-sectional area occupying ratio of the reinforcing wires was 14.9%.

### Comparative Example 2

A 6 Fr guiding catheter was produced by the same method as in Example 1, except that the reinforcing wires had a thickness of 35 *µ*m and a width of 143 *µ*m. In the catheter thus obtained, the width-to-thickness ratio of the braid was 4.09, the ratio of the catheter outside diameter to the width of the reinforcing wires was 58.9, and the cross-sectional area occupying ratio of the reinforcing wires was 14.9%.

### Comparative Example 3

A 6 Fr guiding catheter was produced by the same method as in Example 1, except that the reinforcing wires had a thickness of 40 *µ*m and a width of 143 *µ*m. In the catheter thus obtained, the width-to-thickness ratio of the braid was 4.09, the ratio of the catheter outside diameter to the width of the reinforcing wires was 58.9, and the cross-sectional area occupying ratio of the reinforcing wires was 14.9%.

### Comparative Example 4

A 6 Fr guiding catheter was produced by the same method as in Example 1, except that the reinforcing wires had a thickness of 30 *µ*m and a width of 94 *µ*m. In the catheter thus obtained, the width-to-thickness ratio of the braid was 3.13, the ratio of the catheter outside diameter to the width of the reinforcing wires was 68.7, and the cross-sectional area occupying ratio of the reinforcing wires was 14.9%.

### Comparative Example 5

When a guide catheter Launcher (size: 6 Fr; shape: JL 4.0) distributed by Medtronic Japan, Co., Ltd. was analyzed, the reinforcing wires were found to have a thickness of 40 *µ*m and a width of 110 *µ*m. The interval of the reinforcing wires was 0.3 mm, the width-to-thickness ratio of the reinforcing wires was 2.75, the ratio of the catheter outside diameter to the width of the reinforcing wires was 51.5, and the cross-sectional area occupying ratio of the reinforcing wires was 24.8%.

The catheters obtained in the Examples and Comparative Examples above were subjected to the following tests for determining the kink resistance, flexural rigidity, collapse strength, distal end shape restoring performance, and backup force.

### <Kink resistance evaluation test (Loop method)>

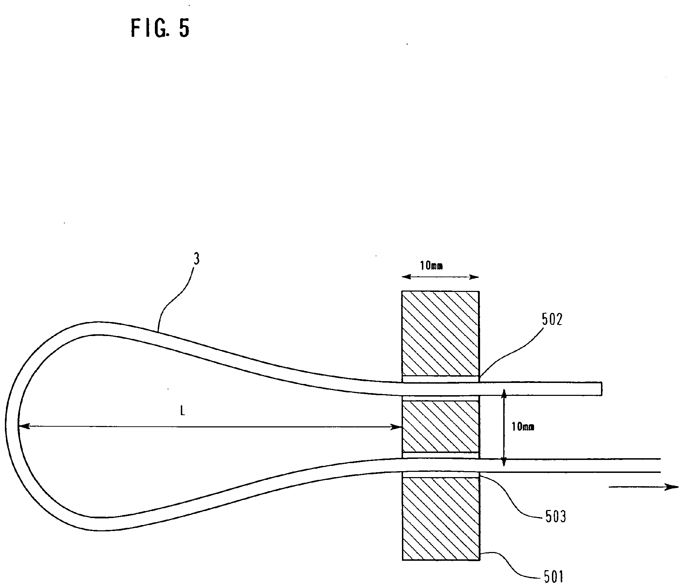

As shown in Fig. 5, a 10 mm-thick plate 501 provided with two holes 502, 503 having a diameter of 2.8 mm, with a center-to-center distance of 10 mm was prepared. The catheter was passed through the two holes 502 and 503 so as to form a loop, one end of the catheter was pulled to contract the loop, and, upon generation of a kink in the loop portion, the distance L between the fold-back end of the loop and the plate 501 was measured. This test was conducted in 37°C warm water, after immersion in 37°C warm water for not less than 30 min.

### <Flexural rigidity evaluation test>

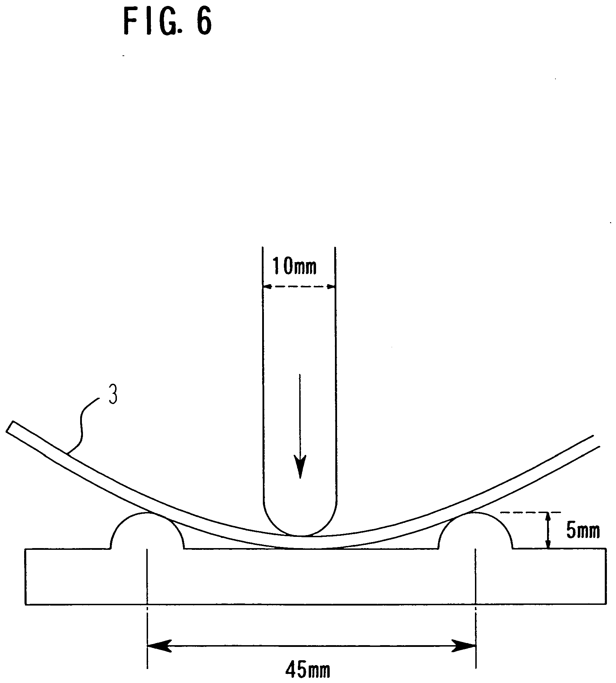

The proximal portion side of the catheter main body portion was immersed in 37°C warm water for not less than 30 min. Thereafter, as shown in Fig. 6, in 37°C warm water, the catheter was put on a stainless steel jig having two 5 mm-high support points with a gauge length of 45 mm, and the stress at the time when a central portion of the catheter was pushed in by 3 mm at a rate of 5 mm/min by use of a pusher having a radius of 5 mm was measured.

### <Collapse strength evaluation test>

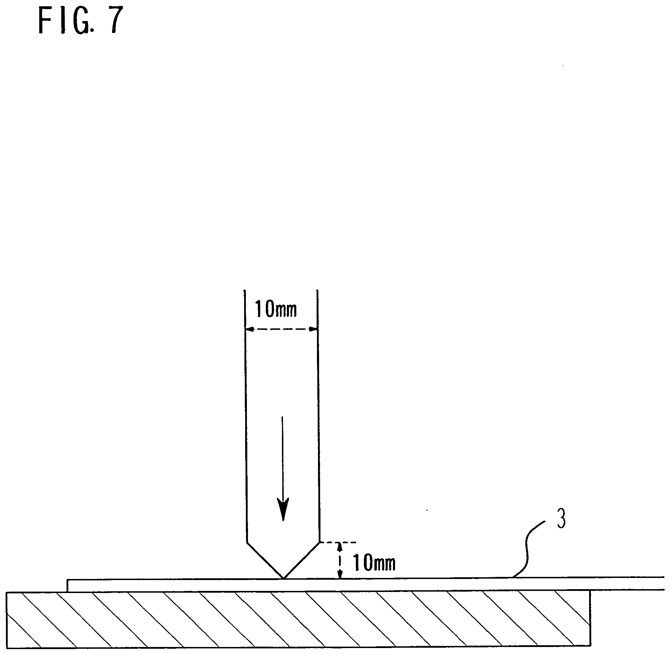

A catheter main body portion was immersed in 37°C warm water for not less than 30 min. Thereafter, as shown in Fig. 7, in 37°C warm water, the push-in force (strength) at the time when the catheter main body portion was collapsed by a pusher with a right-angled tip end was measured. In this test, the collapsed state is defined as a state that the catheter main body portion is pushed by the pusher in 1 mm. The catheter main body portion is not collapsed completely in this state because the inner diameter of the catheter main body portion of this sample is larger than 1mm.

### <Distal end shape restoring performance evaluation test>

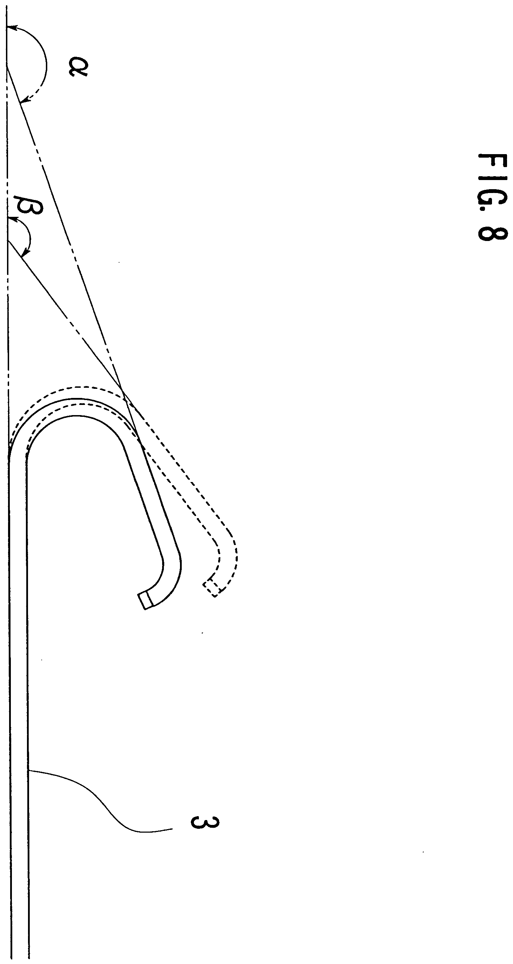

Distal end portions of all the catheters obtained in the Examples and Comparative Examples above were processed into the same JL 4.0 shape as shown in Fig. 8, and the angle *α* of the distal end shaped portion of each catheter was measured. Each catheter put into a rectilinear state was inserted into a sheath, was then immediately evulsed, and, after one minute, the angle *β* was measured. The restoration ratio (%) was determined according to the formula "(*β*/ *α*)×100."

### <Backup force evaluation test>

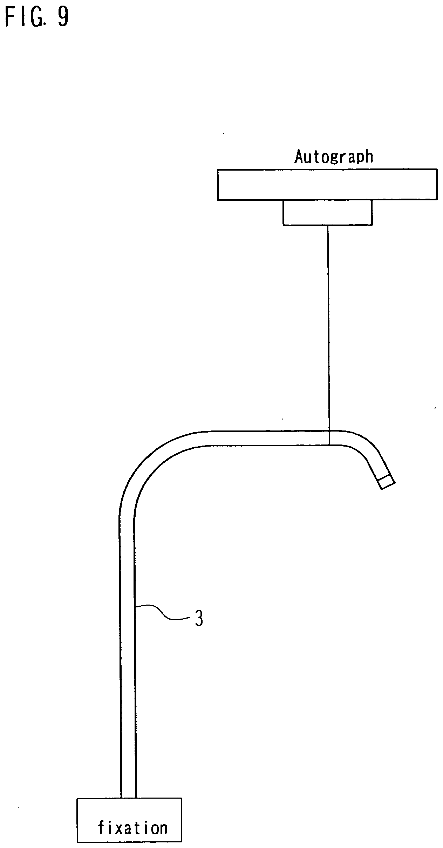

The JL 4.0 shape portion was cut off the catheter, and was immersed in 37°C warm water for not less than 30 min. Thereafter, a proximal end portion of the cut off portion of the catheter was fixed in the hot water, and, as shown in Fig. 9, a thread was attached to a distal end portion of the cut off portion of the catheter. The load at the time when a tension is applied so as to open the curved portion on the proximal side to 90 degrees was measured on an autograph AG-I (produced by Shimadzu Corporation).

The results of the above tests are shown in Table 1 below.

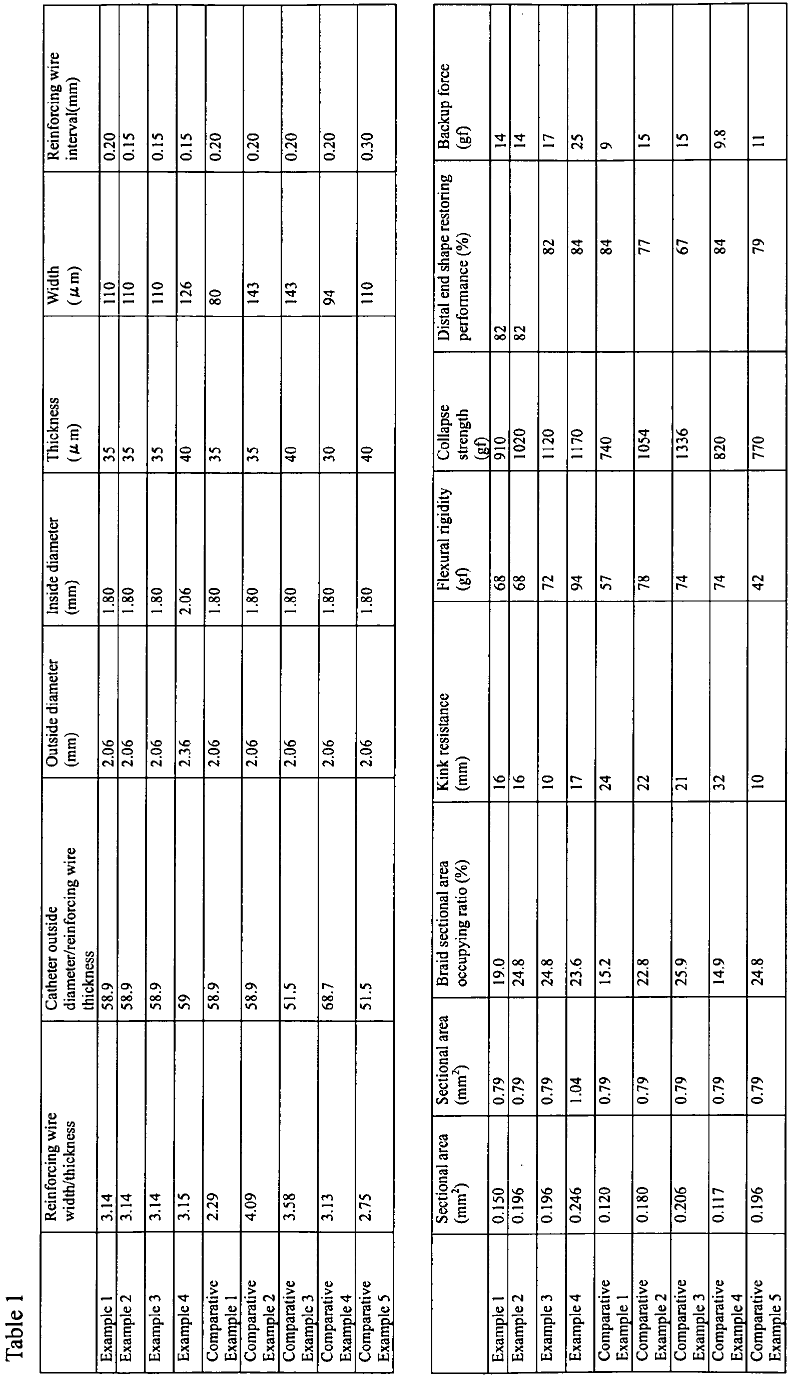

Here, a lower kink resistance value indicates a better kink resistance of the catheter. In addition, a lower flexural rigidity value indicates that the catheter is more flexible. A higher collapse strength value indicates that it is more difficult for the catheter to be broken.

The distal end shape restoring performance is expressed in terms of a value indicating the restoring performance in the case where the catheter is deformed into a shape (inclusive of a rectilinear shape) different from the original shape thereof. The higher the numerical value, the higher (better) the restoring performance and the easier the catheter is to use. A guiding catheter is fed to the coronary ostium from an artery of an arm or leg, for example; therefore, the guiding catheter is once stretched into a rectilinear shape by a sheath or a guide wire, before being inserted into the blood vessel. When the guiding catheter has come close to the coronary ostium, the guide wire or the like is pulled away, and the distal end portion of the guiding catheter is engaged with the coronary ostium. In this case, the distal end portion must be returned to its original shape. If the distal end portion remains in the opened rectilinear shape, the distal end portion is liable to be disengaged from the coronary ostium when a device is inserted into the coronary artery after the distal end portion is engaged with the coronary ostium. Therefore, for quick returning into the original shape, a high distal end shape restoring performance is demanded. A flexible catheter has a high distal end shape restoring performance; while a harder catheter, particularly a catheter having a high cross-sectional area occupying ratio of the reinforcing wires, is lower in distal end shape restoring performance. The distal end shape restoring performance is desirably not less than 80%.

As for the backup force, a higher numerical value indicates that the guiding catheter fixed to the coronary ostium is more stable, and the insertion of a device is easier to carry out. After the guiding catheter is engaged with the coronary ostium, the shape and position of the catheter must be fixed, for easy movements of the device. For example, in the case of the JL shape (Judkins left shape), when the distal end portion is engaged with the left coronary ostium, the second bent portion from the distal end is opened to about 90 degrees. In this case, the catheter is fixed by a repelling force tending to close the catheter opened, with the coronary ostium and the aorta wall as points of support. Therefore, the catheter is fixed more securely as the repelling force is greater. As the backup force value is higher, the force tending to close the shape is greater, and the force (backup force) for fixing the catheter in a clamping manner is enhanced. The backup force is desirably not less than 10 gf.

### <Animal experiment>

When the guiding catheter according to Example 1 was inserted into a pig, which has a meandering iliac artery, via a femoral region by the usual method, the catheter passed smoothly through the meandering portion, was then engaged with the left coronary artery, and manual operations (procedure) were carried out. The operationality of the device was good, and even when a torque was applied by use of the hub, the distal end was rotated, and twisting or kinking was not generated in the catheter.

According to the embodiment, the reinforcing wires are specified, whereby kink resistance is enhanced. Further, with the catheter outside surface subjected to surface roughening or to coating with a lubricating substance, the distal end follows up to rotation on the proximal side even when located in a sharply bent blood vessel. On the other hand, smooth catheters underwent kinking through twisting.

According to the embodiment, a catheter having physical properties optimum for use as a guiding catheter is realized which possesses enhanced kink resistance, is rigid, possesses excellent pushability characteristics at the time of insertion in a meandering blood vessel, is high in distal end shape restoring performance, and is capable of quickly restoring the original shape even after stretched into a straight form. In addition, the catheter according to the present invention has a high backup force, is capable of being engaged with a coronary ostium so securely that it would not easily be disengaged at the time of device operations, has an inside diameter that is relatively large in comparison with the outside diameter, in other words, has a high ratio of inside diameter to outside diameter, and is applicable to use with a variety of devices.

The present invention is not limited to the details of the above-described preferred embodiments. The scope of the invention is defined by the appended claims and all changes and modifications and equivalents falling within the scope of the claims are embraced by the claims.

## Claims

1. A catheter (1) comprising:
an elongated tubular body (3) possessing an outside diameter (D1) of from 1.35 to 3 mm and an inside diameter (d1), the tubular body (3) possessing a cross-sectional area in the direction perpendicular to the longitudinal direction of the catheter main body of (π x [(D1/2)² - (d1/2)²]);
the tubular body (3) comprising a distal end portion (33) and a proximal end portion (31), and being comprised of an inner layer (34) forming an inside surface of said tubular body, an outer layer (35) forming an outside surface of said tubular body, and a plurality of reinforcing wires (361) between said inside surface and said outside surface;
said reinforcing wires (361) each possessing a length, a thickness (d2) substantially parallel to a radial direction of said tubular body (3) and a width substantially perpendicular to said thickness (d2), said plurality of reinforcing wires (361) together possessing a total cross-sectional area;
said tubular body (3) possesses a wall thickness (D2), and a ratio of the wall thickness (D2) of said tubular body (3) to the thickness (d2) of said reinforcing wires (361) is from 3.5 to 3.8;
**characterized in that**
a proportion of the total cross-sectional area of said plurality of reinforcing wires (361) to the cross-sectional area of said tubular body (3) is less than 25%, but not less than 17%.

2. The catheter (1) as set forth in Claim 1, wherein the ratio of the width (w) of said reinforcing wires (361) to the thickness (d2) of said reinforcing wires (361) is more than 2.5 and less than 3.6.

3. The catheter (1) as set forth in Claim 1, wherein a ratio of an outer circumference (π D1) of said tubular body (3) to the width (w) of said reinforcing wires (361) is from 54 to 61.6.

4. The catheter (1) as set forth in Claim 1, wherein a ratio of the outside diameter (D1) of said tubular body (3) to the thickness (d2) of said reinforcing wires (361) is from 55 to 65.

5. The catheter (1) as set forth in Claim 1, wherein the ratio of the inside diameter (d1) to the outside diameter (D1) of said tubular body (3) is from 0.85 to 0.91.

6. The catheter (1) as set forth in Claim 1, wherein said reinforcing wires (361) form an angle (θ) from 65° and 75° relative to a longitudinal direction of the tubular body (3).

7. The catheter (1) as set forth in Claim 1, with an outer diameter of 6 Fr (2.06 mm) and an inner diameter of 1.80 mm.

8. The catheter (1) as set forth in Claim 1, with an outer diameter of 7 Fr (2.36 mm) and an inner diameter of 2.06 mm.

## Patentansprüche

1. Katheter (1), umfassend:
einen langgestreckten rohrförmigen Körper (3), der einen Außendurchmesser (D1) von 1,35 bis 3 mm und einen Innendurchmesser (d1) aufweist, wobei der rohrförmige Körper (3) eine Querschnittsfläche in der Richtung senkrecht zu der Längsrichtung des Katheterhauptkörpers von (π x [(D1/2)² - (d1/2)²]) aufweist;
wobei der rohrförmige Körper (3) einen distalen Endabschnitt (33) und einen proximalen Endabschnitt (31) umfasst und aus einer Innenschicht (34), die eine Innenfläche des rohrförmigen Körpers bildet, einer Außenschicht (35), die eine Außenfläche des rohrförmigen Körpers bildet, und einer Vielzahl von Verstärkungsdrähten (361) zwischen der Innenfläche und der Außenfläche besteht;
wobei die Verstärkungsdrähte (361) jeweils eine Länge, eine Dicke (d2), die im Wesentlichen parallel zu einer radialen Richtung des rohrförmigen Körpers (3) ist, und eine Breite, die im Wesentlichen senkrecht zu der Dicke (d2) ist, aufweisen, wobei die Vielzahl von Verstärkungsdrähten (361) zusammen eine Gesamtquerschnittsfläche aufweisen;
wobei der rohrförmige Körper (3) eine Wanddicke (D2) aufweist und ein Verhältnis von der Wanddicke (D2) des rohrförmigen Körpers (3) zu der Dicke (d2) der Verstärkungsdrähte (361) von 3,5 bis 3,8 beträgt;
**dadurch gekennzeichnet, dass**,
ein Anteil der Gesamtquerschnittsfläche der Vielzahl von Verstärkungsdrähten (361) an der Querschnittsfläche des rohrförmigen Körpers (3) weniger als 25 %, aber nicht weniger als 17 % beträgt.

2. Katheter (1) nach Anspruch 1, wobei das Verhältnis von der Breite (w) der Verstärkungsdrähte (361) zu der Dicke (d2) der Verstärkungsdrähte (361) mehr als 2,5 und weniger als 3,6 beträgt.

3. Katheter (1) nach Anspruch 1, wobei ein Verhältnis eines Außenumfangs (n D1) des rohrförmigen Körpers (3) zu der Breite (w) der Verstärkungsdrähte (361) von 54 bis 61,6 beträgt.

4. Katheter (1) nach Anspruch 1, wobei ein Verhältnis eines Außendurchmessers (D1) des rohrförmigen Körpers (3) zu der Dicke (d2) der Verstärkungsdrähte (361) von 55 bis 65 beträgt.

5. Katheter (1) nach Anspruch 1, wobei das Verhältnis des Innendurchmessers (d1) zu dem Außendurchmesser (D1) des rohrförmigen Körpers (3) von 0,85 bis 0,91 beträgt.

6. Katheter (1) nach Anspruch 1, wobei die Verstärkungsdrähte (361) einen Winkel (θ) von 65° und 75° in Bezug auf eine Längsrichtung des rohrförmigen Körpers (3) bilden.

7. Katheter (1) nach Anspruch 1 mit einem Außendurchmesser von 6 Fr (2,06 mm) und einem Innendurchmesser von 1,80 mm.

8. Katheter (1) nach Anspruch 1 mit einem Außendurchmesser von 7 Fr (2,36 mm) und einem Innendurchmesser von 2,06 mm.

## Revendications

1. Cathéter (1) comprenant :
un corps tubulaire allongé (3) possédant un diamètre extérieur (D1) de 1,35 à 3 mm et un diamètre intérieur (d1), le corps tubulaire (3) ayant une superficie de section transversale dans la direction perpendiculaire à la direction longitudinale du corps principal de cathéter de (π x [(D1/2)² - (d1/2)²]);
le corps tubulaire (3) comprenant une partie d'extrémité distale (33) et une partie d'extrémité proximale (31), et étant composé d'une couche intérieure (34) formant une surface intérieure dudit corps tubulaire, et d'une couche extérieure (35) formant une surface extérieure dudit corps tubulaire, et une pluralité de fils de renforcement (361) entre ladite surface intérieure et ladite surface extérieure ;
lesdits fils de renforcement (361) ayant chacun une longueur, une épaisseur (d2) sensiblement parallèle à une direction radiale dudit corps tubulaire (3) et une largeur sensiblement perpendiculaire à ladite épaisseur (d2), ladite pluralité de fils de renforcement (361) possédant ensemble une superficie de section transversale totale ;
ledit corps tubulaire (3) possède une épaisseur de paroi (D2), et un rapport de l'épaisseur de paroi (D2) dudit un corps tubulaire (3) à l'épaisseur (d2) desdits fils de renforcement (361) va de 3,5 à 3,8 ;
**caractérisé en ce que**
une proportion de la superficie de section transversale totale de ladite pluralité de fils de renforcement (361) à la superficie de section transversale dudit corps tubulaire (3) est inférieure à 25%, mais pas inférieure à 17%.

2. Cathéter (1) selon la revendication 1, dans lequel le rapport de la largeur (w) desdits fils de renforcement (361) à l'épaisseur (d2) desdits fils de renforcement (361) est supérieur à 2,5 et inférieur à 3,6.

3. Cathéter (1) selon la revendication 1, dans lequel un rapport d'une circonférence extérieure (πD1) dudit corps tubulaire (3) à la largeur (w) desdits fils de renforcement (361) va de 54 à 61,6.

4. Cathéter (1) selon la revendication 1, dans lequel un rapport du diamètre extérieur (D1) dudit corps tubulaire (3) à l'épaisseur (d2) desdits fils de renforcement (361) va de 55 à 65.

5. Cathéter (1) selon la revendication 1, dans lequel le rapport du diamètre intérieur (d1) au diamètre extérieur (D1) dudit corps tubulaire (3) va de 0,85 à 0,91.

6. Cathéter (1) selon la revendication 1, dans lequel lesdits fils de renforcement (361) forment un angle (θ) de 65° et 75° par rapport à une direction longitudinale du corps tubulaire (3).

7. Cathéter (1) selon la revendication 1, ayant un diamètre extérieur de 6 Fr (2,06 mm) et un diamètre intérieur de 1,80 mm.

8. Cathéter (1) selon la revendication 1, ayant un diamètre extérieur de 7 Fr (2,36 mm) et un diamètre intérieur de 2,06 mm.
